# EUROPEAN PATENT SPECIFICATION

(11) **EP 4 534 002 B1**
(45) Date of publication and mention of the grant of the patent: **01.04.2026**
(21) Application number: 24204043.4
(22) Date of filing: 01.10.2024
(51) Int. Cl.: A61B 5/00, A61C 9/00, G06T 5/60, G06T 17/00, G06T 5/00, G06T 7/521

(54) **INTRAORAL SCANNER SYSTEM AND METHOD FOR REMOVING ARTIFACTS ARISING FROM REFLECTIONS**
NTRAORALES SCANNERSYSTEM UND VERFAHREN ZUR ENTFERNUNG VON ARTEFAKTEN DURCH REFLEXIONEN
SYSTÈME DE SCANNER INTRAORAL ET PROCÉDÉ D'ÉLIMINATION D'ARTEFACTS PROVENANT DE RÉFLEXIONS

(30) Priority: 04.10.2023 DK PA202370507
(43) Date of publication of application: 09.04.2025
(73) Proprietor: 3Shape A/S, 1060 Copenhagen K (DK)
(72) Inventor: BILBERG, Christopher Prinds, 1060 Copenhagen K (DK); MOTTELSON, Isak, 1060 Copenhagen K (DK)
(74) Representative: Zacco Denmark A/S

(56) References cited:
- WO-A1-2020/264035
- WO-A1-2023/012792
- WO-A1-2023/117981

## Description

### Technical field

The present disclosure relates to a computer-implemented method for improving the accuracy of a three-dimensional (3D) representation. The present disclosure further relates to a 3D scanner system comprising one or more processors configured for performing the steps of the method disclosed herein. The disclosure further relates to a sleeve for an intraoral scanner.

### Background

3D scanning technology is widely used in dentistry for creating a digital impression of teeth. This technology enables dentists to obtain a detailed and accurate representation of the teeth in a digital format, which can then be used for a variety of applications, including the design and fabrication of dental restorations, orthodontic appliances, and other dental prostheses.

Typically, the digital impression is generated using an intraoral scanner maneuvered inside the mouth of a patient. An important aspect hereof is hygiene, since the intraoral scanner comes into contact with the patient. Some existing solutions utilize sterilized tips of the scanner, e.g., sterilized using an autoclave, to ensure a hygienic distal end of the scanner. Other suggested solutions utilize single-use tips, i.e., scanning tips adapted to be used only on a single patient. Yet other solutions utilize a sheath for a tip of the scanner, such as the sheath disclosed in WO 2020/053136 A1 by 3Shape A/S.

WO2020264035A1 discloses a method for generating a 3D image includes driving structured light projector(s) to project a pattern of light on an intraoral 3D surface, and driving camera(s) to capture images, each image including at least a portion of the projected pattern, each one of the camera(s) comprising an array of pixels. A processor compares a series of images captured by each camera and determines which of the portions of the projected pattern can be tracked across the images. The processor constructs a three-dimensional model of the intraoral three-dimensional surface based at least in part on the comparison of the series of images. Other embodiments are also described.

However, a problem associated with all of the existing solutions is that the scanning tips and/or the sheath typically give rise to a number of undesired reflections in the captured images by the intraoral scanner. The reflections are often caused by e.g. specular reflection(s) of light projected by the scanner. These undesired reflections cause artifacts in the images. Sometimes, in case a structured pattern is projected by the scanner, the pattern is reproduced in the images as an echo, which complicates the image processing performed by the 3D scanner system. Consequently, the accuracy of the generated digital impression may suffer or in some cases false 3D points are generated based on the artifacts.

Therefore, there is a need for improved methods and systems for removing artifacts arising from reflections, in particular within applications using 3D scanner systems or intraoral scanners. Furthermore, there is a need for new and improved hygienic solutions for intraoral scanning applications.

### Summary

The solution is defined in the independent system claim 1, and advantageous embodiments are defined in the dependent claims. Typically, an intraoral scanner utilizes structured light projected onto the dentition of the patient in order to generate a 3D model of the dentition. Since an intraoral scanner is a medical device intended for use within the oral cavity of the patient, it is important to ensure hygiene and cleanliness of the device. As mentioned, existing solutions typically either use single use scanning tips, autoclavable scanning tips, or in some cases a sleeve covering the scanner tip. All the mentioned solutions introduce a transparent hygienic barrier in the optical path of the scanner, i.e. in a path of the projected light from the scanner. A problem associated herewith is that the barrier gives rise to some unwanted reflections; in particular specular reflections caused by the projected light from the scanner. The specular reflections from the barrier are unwanted as they complicate the task of determining the 3D topography of the scanned object. Furthermore, most scanners rely on capturing the reflected light from the scanned object including a projected pattern on the object. If the pattern, or a part of the pattern, is reflected by the hygienic barrier, the scanner captures an 'echo' of the reflected pattern, which is unwanted, since it is not related to the surface of the scanned object. Thus, the captured images may comprise artifacts arising from the reflections from the hygienic barrier, which similarly complicates the generation of 3D points of the scanned surface.

The present disclosure solves the above-mentioned challenges by providing a 3D scanner system comprising:
- an intraoral scanner comprising:
   - an elongated housing comprising a distal end for being inserted into an oral cavity, wherein the housing comprises an aperture in a sidewall of the distal end of the housing; and
      - a window arranged in the aperture of the housing; and/or
      - a sleeve mounted on the outside of the elongated housing, wherein the sleeve covers the aperture;
- one or more processors operatively connected to the intraoral scanner, said processors configured to:
   - receive one or more two-dimensional images comprising a plurality of pattern features, wherein the images comprises one or more artifacts arising from reflections from the window and/or sleeve; and
   - provide the two-dimensional image(s) as input to a parameterized model trained to determine the position of at least a subset of the pattern features in the image(s), while suppressing or ignoring the artifacts.

In preferred embodiments, one or more parameters of the parameterized model have been chosen or adjusted such that the artifacts in the images are suppressed or ignored by the parameterized model. The parameterized model may be a machine learning model, such as a neural network. In some embodiments, the parameterized model is a convolutional neural network trained to suppress or ignore the artifacts and/or ignore the part of the spatial pattern which has been reflected by the window and/or sleeve.

The present inventors have realized that a trained parameterized model is useful for achieving the desired outcome of ignoring the artifacts, such that 3D points are determined more accurately, and/or such that less false 3D points are generated. In particular, the present inventors have realized how to train the parameterized model to ignore the artifacts, e.g., how to generate the training data, what model and architecture to choose, etc. It is advantageous to split the problem in two, such that there is some data for which the ground truth is known and other data representing only the artifacts. In this case, the ground truth may be understood as the true position of the pattern features in the images, said pattern features forming part of the projected pattern by the scanner. The ground truth may be generated by rendering 3D models of previously scanned objects or by using real 3D data of scanned objects without any window or sleeve. The rendered images may have been generated using a geometric virtual model of the intraoral scanner, wherein said geometric virtual model includes extrinsic and/or intrinsic parameters of the projector unit and camera units of the scanner.

The remaining part of the training data may advantageously be two-dimensional training images, said images ideally only containing the artifacts. These training images may have been acquired through the window and/or sleeve, wherein there is no object in focus except for the window and/or the sleeve. This ensures that only the window / sleeve reflections are recorded in the training images. This approach is one example of how to ensure that the foreground (represented by the window/sleeve surface) can be separated from the background, e.g., the images of the scanned surface.

The present disclosure further relates to a computer-implemented method comprising the steps of:
- receiving one or more two-dimensional images acquired by one or more camera units forming part of an intraoral scanner, wherein each image comprises at least a part of a spatial pattern comprising a plurality of pattern features, wherein the image(s) further comprises one or more artifacts arising from reflections from a window and/or sleeve of the intraoral scanner; and
- providing the two-dimensional image(s) as input to a parameterized model configured to determine the position of at least a subset of the pattern features in the image(s), wherein one or more parameters of the parameterized model is chosen such that the artifacts arising from reflections from the window and/or sleeve are suppressed or ignored.

Any of the embodied computer-implemented methods disclosed herein may be performed, either fully or partly, by the 3D scanner system disclosed herein. The present disclosure further relates to a data processing system, such as a 3D scanner system, comprising one or more processors configured for performing the steps of the disclosed method. The present disclosure further relates to a computer program product comprising instructions which, when the program is executed by a computer, cause the computer to carry out the steps of the disclosed computer-implemented method. The computer may form part of the 3D scanner system. The present disclosure further relates to a computer-readable data carrier having stored thereon said computer program product.

In another aspect, the present disclosure relates to a 3D scanner system comprising:
- an intraoral scanner comprising:
   - an elongated housing comprising a distal end for being inserted into an oral cavity, wherein the housing comprises an aperture in a sidewall of the distal end of the housing; and
      - a window arranged in the aperture of the housing; and/or
      - a sleeve mounted on the outside of the elongated housing, wherein the sleeve covers the aperture;
- one or more processors operatively connected to the intraoral scanner, said processors configured to:
   - receive one or more two-dimensional images comprising a plurality of pattern features and one or more artifacts arising from reflections from the window and/or sleeve; and
   - subtract, from each of the images, a previously obtained background image, wherein said image has been obtained with no object in focus except for the window and/or sleeve, whereby modified two-dimensional images with a reduced number of artifacts are obtained; and
   - generate a digital three-dimensional representation based on the modified two-dimensional images.

The previously obtained background image may represent the window and/or sleeve with no object behind, i.e., with a black background. In that way, the reflections/artifacts are directly recorded in the background image, which subsequently can be subtracted from the received two-dimensional images of the scanned object to remove, ideally all, the artifacts.

Accordingly, the presently disclosed system and method enables artifacts arising from window and/or sleeve reflections to be suppressed or ignored, whereby a more accurate 3D model can be generated of the scanned object.

### Brief description of the drawings

Fig. 1 shows a flowchart according to an embodiment of the computer-implemented method disclosed herein.
Fig. 2 shows an embodiment of a 3D scanner system according to the present disclosure.
Fig. 3 shows a flowchart according to an embodiment of the computer-implemented method disclosed herein.
Fig. 4 shows a computer system in which embodiments of the present disclosure, or portions thereof, may be implemented as computer-readable code.
Fig. 5 shows an intraoral scanner and a sleeve according to an embodiment of the present disclosure.
Fig. 6 shows an intraoral scanner and a sleeve according to an embodiment of the present disclosure.
Fig. 7A shows a single sub-scan (3D representation) generated by the scanner in a case where the parameterized model has not been trained to ignore or suppress artifacts.
Fig. 7B shows a few subscans that are fused into a 3D model that resembles a noisy plane, or noisy displaced elements of a plane.
Fig. 8A shows a raw image of some teeth, wherein the image has been captured through a window arranged in the aperture of the housing of an intraoral scanner.
Fig. 8B shows a digital 3D model of the same teeth shown in fig. 7A, wherein the 3D model has been generated based on a plurality of captured images of the teeth.
Fig. 9A shows a raw image of some teeth, wherein the image has been captured through a window and a sleeve, wherein the sleeve covers the window.
Fig. 9B shows a rendition of the digital 3D model, where the images have been captured through both a window and a sleeve covering the window, wherein the parameterized model has been trained to ignore or suppress artifacts from the window and/or sleeve.

### Detailed description

### Intraoral scanner

The scanner disclosed herein may be an intraoral scanner for acquiring images within an intraoral cavity of a subject. The scanner may be a handheld scanner, i.e. a device configured for being held with a human hand. The scanner may employ any suitable scanning principle such as focus-based scanning, triangulation-based scanning, stereo vision, structure from motion, confocal scanning, or other scanning principles.

In some embodiments, the scanner employs a triangulation-based scanning principle. As an example, the scanner may comprise a projector unit and one or more camera units for determining points in 3D space based on triangulation. As another example, the scanner comprises a projector unit and two or more camera units, wherein the camera units are configured to image the scanned object from separate views, i.e. from different directions. In particular, the camera units may be configured to acquire a set of images, wherein a correspondence problem is solved within said set of images based on triangulation. The images within the set of images may be acquired by separate camera units of the scanner.

A triangulation-based intraoral scanner is further described in the following applications by the same applicant: WO 2023/117981 A1 "Systems and methods for generating a digital representation of a 3D object" filed on 19 December 2022, PCT/EP2023/058521 "Intraoral 3D scanning device for projecting a high-density light pattern" filed on 31 March 2023, and PCT/EP2023/058980 "Intraoral scanning device with extended field of view" filed on 5 April 2023.

The intraoral scanner may comprise one or more scan units, wherein each scan unit comprises a projector unit and one or more camera units. As an example, the scanner may comprise one scan unit comprising one projector unit and at least two camera units. As another example, the scanner may comprise one scan unit comprising one projector unit and four camera units. In yet another example, the scanner may comprise at least two scan units, wherein each scan unit comprises a projector unit and two or more camera units. In yet another example, the scanner may comprise at least two scan units, wherein each scan unit comprises a projector unit and four camera units. The scan units may in some embodiments be arranged in series to enable an extended or enlarged field of view (FOV) of the intraoral scanner. Thus, each scan unit may provide a FOV, such that two scan units provides a combined FOV of approximately double the FOV of a single scan unit.

### Housing

The intraoral scanner may comprise an elongated housing comprising a distal end for being inserted into an oral cavity. The housing may be configured to accommodate a number of optical and/or electronic components of the scanner. The housing may constitute a rigid outer shell of the scanner. The housing may comprise an aperture located in a sidewall of the distal end of the housing. The aperture is preferably arranged such that it is located in an optical path of the light projected by the scanner. In some embodiments, the housing of the scanner comprises two or more parts, such as a fixed part and a replaceable part, which is configured for being replaceably attached to the fixed part of the housing. As an example, the scanner may comprise a replaceable scanning tip configured for being mounted to the scanner or elongated housing. In that case, the scanning tip may comprise an aperture or window configured to align with a corresponding aperture or window of the elongated housing.

In some embodiments, the intraoral scanner comprises a window arranged in the aperture of the housing and/or of the scanning tip. Thus, the scanner may comprise a window arranged in the aperture of the housing, wherein the projector unit and window are arranged such that the pattern is projected through the window during use of the scanner. In preferred embodiments, at least a region of the window is made of a transparent material, said region located in an optical path of the projected spatial pattern. The window may be made of a polymer, such as poly(methyl methacrylate) (PMMA), or a glass material, such as Sapphire glass. The window is preferably rigid and planar. In some embodiments, the window is coated with an antireflective (AR) coating. The window may be located in an optical path of the scanner, i.e., located such that the projected pattern of light is projected through the window during use of the scanner.

### Projector unit

The intraoral scanner may comprise one or more projector units. Each projector unit may be configured to project a predefined spatial pattern through the aperture and onto at least a part of the surface of a three-dimensional object.

A projector unit may be understood herein as a device configured for projecting light onto a surface, such as the surface of a three-dimensional object. In preferred embodiments, the projector unit is configured to project a pattern of light, such as the spatial pattern, onto the surface of a three-dimensional object, such as a dental object. Preferably, the projector unit is configured to project a pattern of light such that the spatial pattern is, within a given tolerance, in focus at a predefined focus distance or range, when measured along a projector optical axis. Thus, the spatial pattern may be in an acceptable focus within a given focus range. The projector unit may be configured to project unpolarized light.

The projector unit may be selected from the group of: Digital Light Processing (DLP) projectors using a plurality of digital micromirror devices (DMD) arranged in a matrix or array, or diffractive optical elements (DOE), or front-lit reflective mask projectors, or micro-LED projectors, or Liquid crystal on silicon (LCoS) projectors or back-lit mask projectors, wherein a light source is placed behind a mask having a spatial pattern. The pattern may be dynamic, i.e. such that the pattern changes over time, or the pattern may be static in time, i.e. such that the pattern remains the same over time. An advantage of projecting a static pattern is that it allows the capture of all the image data simultaneously, thus preventing warping due to movement between the scanner and the object.

The projector unit may comprise one or more collimation lenses for collimating the light from the light source. The collimation lens(es) may be placed between the light source and a mask in a back-lit mask projector unit. In some embodiments, the one or more collimation lenses are Fresnel lenses. The projector unit may further comprise one or more focus lenses configured for focusing the projected spatial pattern at a predefined focus range. The projector focus lenses may define a projector optical axis.

In preferred embodiments, the projector unit of the scanner comprises at least one light source and a pattern generating element for defining a pattern of light. The pattern generating element is preferably configured for generating a light pattern to be projected on a surface of an object. As an example, the pattern generating element may be a mask having a spatial pattern. Hence, the projector unit may comprise a mask configured to define a pattern of light. The mask may be placed between the light source of the projector unit and the one or more focus lenses, such that light transmitted through the mask is patterned into a spatial pattern. As an example, the mask may define a polygonal pattern comprising a plurality of polygons, such as a checkerboard pattern. In other embodiments, the pattern generating element is based on diffraction and/or refraction to generate the light pattern, such as a pattern comprising an array of discrete unconnected dots.

Preferably, the projector unit is configured to generate a predefined static pattern, which may be projected onto a surface. Alternatively, the projector unit may be configured to generate a dynamic pattern, which changes in time, i.e., a time-varying pattern. The projector unit may be associated with its own projector plane, which is determined by the projector optics. As an example, if the projector unit is a back-lit mask projector, the projector plane may be understood as the plane wherein the mask is contained. The projector plane comprises a plurality of pattern features of the projected pattern.

### Light source

The projector unit may comprise one or more light sources. The projector unit may be configured to project a spatial pattern of light defined by a plurality of projector rays when the light source(s) are on/active. The projector unit may be configured for sequentially turning the light source on and off at a predetermined frequency, wherein the light source is on for a predetermined time period. The light source(s) may be configured to generate light of a single wavelength or a combination of wavelengths (mono- or polychromatic). The combination of wavelengths may be produced by a light source configured to produce light comprising different wavelengths, or a range of wavelengths (such as white light). The light source may be configured to generate unpolarized light, such as unpolarized white light.

In some embodiments, each projector unit comprises a light source for generating white light. An advantage hereof is that white light enables the scanner to acquire data or information relating to the surface geometry and to the surface color simultaneously. Consequently, the same set of images can be used to provide both geometry of the object, e.g. in terms of 3D data / a 3D representation, and color of the object. Hence, there is no need for an alignment of data relating to the recorded surface geometry and data relating to the recorded surface color in order to generate a digital 3D representation of the object expressing both color and geometry of the object. Alternatively, the projector unit may comprise multiple light sources such as LEDs individually producing light of different wavelengths (such as red, green, and blue) that may be combined to form light comprising different wavelengths. Thus, the light produced by the light source(s) may be defined by a wavelength defining a specific color, or a range of different wavelengths defining a combination of colors such as white light. In some embodiments, the light source is a diode, such as a white light diode, or a laser diode. In some embodiments, the projector unit comprises a laser, such as a blue or green laser diode for generating blue or green light, respectively. An advantage hereof is that a more efficient projector unit can be realized, which enables a faster exposure compared to utilizing e.g. a white light diode.

In some embodiments, the scanner comprises a light source configured for exciting fluorescent material to obtain fluorescence data from the dental object such as from teeth. Such a light source may be configured to produce a narrow range of wavelengths. In other embodiments, the scanner comprises one or more infrared light sources, configured to generate wavelengths in the infrared range, such as between 700 nm and 1.5 µm. In some embodiments, the scanner comprises one or more light sources selected from the group of: Infrared (IR) light source, near-infrared (NIR) light source, blue light source, violet light source, ultraviolet (UV) light source, and/or combinations thereof. In some embodiments, the scanner comprises a first light source forming part of the projector unit, and one or more second light sources, e.g. IR-LED(s) or NIR-LED(s) and/or blue or violet LED(s), located in a distal part of the scanner, such as in the tip of the scanner. Some of the light sources may be utilized for diagnostic purposes, such as for aiding in the detection of regions of caries or plaque.

### Pattern of light

The projector unit may be configured to project a spatial pattern of light defined by a plurality of projector rays when a light source of the projector unit is turned on. The spatial pattern may comprise a plurality of pattern features. In some embodiments, the pattern features are arranged in a two-dimensional (2D) grid. The terms 'illumination pattern', 'pattern of light', 'spatial pattern', and 'projected pattern' are used herein interchangeably.

The spatial pattern may be generated using a pattern generating element, e.g. located in the projector unit. The pattern generating element may be a mask, such as a transparency or transmission mask, having a spatial pattern. The mask may be a chrome photomask. In other embodiments, the pattern generating element is configured to utilize diffraction and/or refraction to generate a light pattern. The use of a pattern of light may lead to a correspondence problem, where a correspondence between points in the light pattern and points seen by the camera unit(s) viewing the pattern needs to be determined. In some embodiments, the correspondence problem is solved jointly for groups of projector rays emanating from the projector unit.

The spatial pattern may be a polygonal pattern comprising a plurality of polygons. The polygons may be selected from the group of: triangles, rectangles, squares, pentagons, hexagons, and/or combinations thereof. Other polygons can also be envisioned. In general, the polygons are composed of edges and corners. In preferred embodiments, the polygons are repeated in the pattern in a predefined manner. As an example, the pattern may comprise a plurality of repeating units, wherein each repeating unit comprises a predefined number of polygons, wherein the repeating units are repeated throughout the pattern. Alternatively, the pattern may comprise a predefined arrangement comprising any one or more of: stripes, squares, dots, triangles, rectangles, and/or combinations thereof. In some embodiments, the pattern is non-coded, such that no part of the pattern is unique.

In some embodiments, the generated pattern of light is a polygonal pattern, such as a checkerboard pattern comprising a plurality of checkers. Similar to a common checkerboard, the checkers in the pattern may have alternating dark and bright areas corresponding to areas of low light intensity (dark) and areas of high(er) light intensity (bright). In some embodiments the pattern of light is a checkerboard pattern comprising alternating squares of dark and bright light. In other embodiments, the light pattern comprises a distribution of discrete unconnected spots of light.

The spatial pattern preferably comprises a plurality of pattern features. The pattern features may be arranged in a regular grid. In some embodiments of the presently disclosed scanner, the total number of pattern features in the pattern is at least 1000, preferably at least 3000, more preferably at least 10000, even more preferably at least 15000. When projecting a pattern comprising such pattern features onto a surface of the 3D object, the acquired images of the object will similarly comprise a plurality of pattern features corresponding to the projected pattern features. A pattern feature may be understood as an individual well-defined location in the pattern or in the image. Examples of pattern features include corners, edges, vertices, points, transitions, dots, stripes, etc. In preferred embodiments, the pattern features comprise the corners of checkers in a checkerboard pattern. In other embodiments, the pattern features comprise corners in a polygon pattern such as a triangular pattern.

### Camera unit

The intraoral scanner may comprise one or more camera units configured to acquire one or more two-dimensional images of the three-dimensional object. Preferably, at least a part of the spatial pattern is present in the images. A camera unit may be understood herein as a device for capturing an image of an object. Each camera unit may comprise an image sensor for generating an image based on incoming light e.g. received from an illuminated 3D object. As an example, the image sensor may be an electronic image sensor such as a charge-coupled device (CCD) or an active-pixel sensor (CMOS sensor). In some embodiments, the image sensor is a global shutter sensor configured to expose the entire image area (all pixels) simultaneously and generate an image in a single point in time. In other embodiments, the image sensor is a rolling shutter sensor.

The image sensor(s) may comprise an array of pixels, wherein each pixel is associated with a corresponding camera ray. The array of pixels may be a two-dimensional (2D) array. Each pixel may be covered by a micro lens. In some embodiments, the image area, i.e. the 2D array of pixels, is rectangular or quadratic. In some embodiments, the image sensor is a CMOS sensor comprising an analog-to-digital converter (ADC) for each column of pixels, making conversion time significantly faster and allowing each camera unit to benefit from greater speed. Each image sensor may define an image plane, which is the plane that contains the object's projected image. Each image obtained by the image sensor(s) may comprise a plurality of pattern features originating from a projected pattern comprising a plurality of such pattern features. In some embodiments, one or more of the camera units comprise a light field camera. Preferably, each camera unit defines a camera optical axis. The camera units may further comprise one or more focus lenses for focusing light.

In some embodiments, the image sensor is a monochrome image sensor, wherein each pixel is associated with a single color channel, e.g. is a grayscale color channel, wherein the value of each pixel represents only an amount of light. In other embodiments, the image sensor is a color image sensor or an image sensor comprising a color filter array on the array of pixels. As an example, the color filter array may be a Bayer filter employing an arrangement of four color filters: Red (R), Green (G), Green (G), and Blue (B). The Bayer filter may also be referred to as an RGGB filter. When utilizing the image sensor data, color pixels may be combined to monochrome pixels of 2 x 2 color pixels for 3D depth reconstruction. In this case, the resolution of the 3D depth reconstruction is only half the resolution of the image sensor in each direction. When obtaining texture (color) images the full native resolution is preferably utilized (with color filtered pixels).

In accordance with some embodiments, the projector optical axis and the camera optical axis, or axes, are non-parallel. As an example, the projector optical axis and the camera optical axis of at least one camera unit may define a camera-projector angle of approximately 5 to 15 degrees, preferably approximately 5 to 10 degrees. All of the camera units may be angled similarly with respect to the projector unit, such that each camera optical axis defines approximately the same angle with the projector optical axis. In some embodiments, the camera units are defocused at the opening of the probe of the scanner and/or at the surface of an optical window in said probe. In preferred embodiments of the scanner, the camera units and projector unit of a given scan unit are focused at the same distance.

Each camera unit may comprise one or more focus lenses for focusing light onto the image sensor of the given camera unit. In some embodiments, each camera unit comprises two or more lenses assembled in a camera lens stack. The purpose of the focus lenses may be to define or ensure a predetermined focus distance, or working distance, of the camera unit. The camera focus lenses may further define the camera optical axis.

In some embodiments, the scanner comprises two or more camera units configured for acquiring a set of images comprising at least one image from each camera unit, wherein each image includes at least a portion of the projected pattern. In preferred embodiments, the images within the set of images are acquired simultaneously. Furthermore, the number of images in the set of images may preferably correspond to the number of camera units, wherein each camera unit contributes one image to the set of images. An advantage hereof, is that the light-budget is improved; thus, less power is consumed by the light source and the projector unit. Consequently, less heat is generated by said components, which is desired, since oftentimes it is difficult to remove heat from intraoral scanners.

The 3D scanner system may comprise one or more processors configured to generate a 3D representation based on the set of images, e.g. by identifying pattern features in the set of images and determining points in 3D space based on triangulation. Some of the processors may be located on the scanner and/or some may be located on an external computer. The 3D representation may be generated continuously during a scanning session, and/or it may be generated in real-time. The 3D scanner system may further comprise a display for displaying the 3D representation. The rendering of the 3D representation and the display of said representation may further occur in real-time, or perceived real-time to the user.

### 3D representation

The 3D scanner system may comprise one or more processors operatively connected to the intraoral scanner, wherein said processors are configured to generate a 3D representation of a scanned object. A 3D representation may be understood herein as a digital representation of a three-dimensional object's external geometry and shape. The 3D representation may represent only a part of the object's geometry and shape. The 3D representation may comprise a collection of points and/or polygons that collectively define the object's surface. As an example, the 3D representation may be selected from the group of: a point cloud, a signed distance field, a triangulated point cloud, a collection of point clouds optionally with additional information such as uncertainty estimates or color(s), a collection of triangulated point clouds, a polygon mesh, a volumetric representation such as a voxel model, a parametrized surface, a surface elements model, or any other suitable three-dimensional representational model. In the 3D representation, each point may be defined by its three-dimensional coordinates (x, y, z) in a Cartesian coordinate system. The points may form part of a surface mesh, such as a polygon mesh. In some embodiments, the 3D representation is a triangle mesh comprising a set of triangles connected by their common edges or vertices. The collection of points and/or the surface mesh may be used to create a visual representation of the object, which can be rendered on a computer screen.

The 3D representation may be generated from a plurality of 3D frames. A 3D frame may be understood herein as a 3D representation generated from a single field of view of an intraoral scanner. In other words, a 3D frame may in itself constitute a 3D representation of a part of the scanned object's surface, and thus may fall within the examples of 3D representations given above. As an example, each 3D frame may constitute a point cloud having a plurality of points in three-dimensional space. During a scan, the scanner may acquire 3D frames at a given frame rate, such as between 20 to 35 frames per second. The 3D frames may be registered in a common reference system and stitched together to form a 3D representation, which is larger than what can be captured in a single field of view of the scanner. Registration of 3D frames may include registering a 3D frame to one or more previously captured 3D frames of the object. Registration of a 3D frame may be understood as determining the position and/or the orientation of the 3D frame relative to another object and/or collection of objects (such as a collection of 3D frames). The registration may further include the actual positioning of the 3D frame relative to the collection of objects and/or collection of 3D frames. A collection of 3D frames stitched together may constitute the digital 3D representation described herein above. The 3D representation, e.g., of a given jaw or of a part of the dental arch, may be composed from a large number of 3D frames, such as between 100 and 2500 frames. Other amounts of 3D frames can be envisioned without departing from the scope of the disclosure.

### Sleeve

The 3D scanner system may comprise a sleeve mounted on the scanner, such as mounted on the outside of the elongated housing. Preferably, the projector unit and the sleeve are arranged such that the pattern is projected through the sleeve during use of the scanner, wherein at least a part of the spatial pattern is reflected by the window and/or the sleeve. Accordingly, the sleeve may, when mounted on the scanner, be configured to cover at least the aperture and/or the window of the scanner.

In some embodiments, the sleeve is made of a silicone material or a polymer, such as a thin polymer material. As an example, the sleeve may be made of a material selected from the group of: polyurethane (PU), polyethylene (PE), polypropylene (PP), low-density polyethylene (LDPE), polyvinyl Chloride (PVC), thermoplastic elastomers (TPE), polyethylene terephthalate (PET), fluoropolymers (e.g., PTFE), polyolefin-based materials, or silicone materials. The selected material may be a material suitable for medical devices. The sleeve may be made in a thin and flexible plastic material.

The sleeve may be flexible, i.e. it may be made of a flexible material. In some embodiments, the entirety of the sleeve is flexible; in other embodiments at least a part of the sleeve is flexible. The flexibility may ensure a tight fit around the housing of the intraoral scanner. In particular, it is preferred if the sleeve is configured such that the sleeve forms a tight and planar surface at least in a region close to the aperture or window of the scanner. Since wrinkles in the sleeve typically cause many reflections, it is desired to mount the sleeve in a straight and tight manner across the aperture or window. In some embodiments, there is no gap between the sleeve and the window; however, in some cases a small gap may be present. In other embodiments, the sleeve is a rigid sleeve, e.g., obtained from injection-molding. The rigid sleeve may comprise a transparent region configured to align with the aperture of the housing, when the sleeve is mounted on the intraoral scanner. Similar to the flexible sleeve, the rigid sleeve may be configured to ensure a close fit around the housing of the scanner. In some embodiments, a first part of the sleeve is rigid and a second part is flexible. As an example, a distal part of the sleeve may be rigid, or semi-rigid, with a flexible part extending from the rigid part.

The sleeve is preferably transparent, at least in a region located in an optical path of the projected spatial pattern, e.g., said region approximately coinciding with the region of the aperture or window of the scanner. In some embodiments, the entirety of the sleeve is transparent, at least to visible light, such as white light. Preferably, the sleeve is transparent at least to wavelengths within visible light, infrared light, and/or ultraviolet light.

The sleeve may be made in a thin material, such as a thin film. The sleeve may have a thickness from about 200 µm to about 800 µm, such as from about 300 µm to about 600 µm, such as from about 400 µm to about 500 µm. The sleeve may have a substantially even thickness everywhere, or it may have a thinner thickness in the region located in the optical path of the projected spatial pattern, when the sleeve is mounted on the scanner, wherein thinner is understood as relative to other regions of the sleeve.

The sleeve is preferably closed in a distal end for covering the distal end of the elongated housing. Accordingly, the sleeve may comprise only one opening for inserting the intraoral scanner. The sleeve may resemble an elongated bag having substantially the same shape as the housing of the scanner. To ensure hygiene when utilizing the intraoral scanner, the sleeve preferably covers a majority of the elongated housing, when mounted on the intraoral scanner. In some embodiments, the sleeve covers, when mounted on the scanner, at least 80 % of the housing, preferably at least 90 % of the housing, even more preferably at least 95 % of the housing. In some cases, the proximal end of the scanner is not covered by the sleeve, since the ventilation ducts for cooling the scanner are sometimes placed in this end.

### Method outlined

A first step of the computer-implemented method disclosed herein may be to receive or obtain one or more two-dimensional images acquired by one or more camera units forming part of an intraoral scanner. Each of the two-dimensional images may comprise at least a part of a spatial pattern comprising a plurality of pattern features. The spatial pattern may be projected on a scanned object by a projector unit forming part of the intraoral scanner. The images may further comprise one or more artifacts arising from reflections from a window and/or sleeve of the intraoral scanner.

A second step of the computer-implemented method disclosed herein may be to provide the two-dimensional image(s) as input to a parameterized model configured to determine the position of at least a subset of the pattern features in the image(s). In preferred embodiments, one or more parameters of the parameterized model is chosen such that the artifacts arising from reflections from the window and/or sleeve are suppressed or ignored by the parameterized model.

A third step of the computer-implemented method disclosed herein may be to generate a digital three-dimensional representation of the scanned object based on the determined position of the pattern features. This may be achieved by triangulating projector rays and camera rays corresponding to the pattern features. Thus, the digital 3D representation may be generated based on a triangulation principle.

Any of the computer-implemented methods disclosed herein may be executed, either fully or partly, by one or more processors forming part of a 3D scanner system. The processors may further be operatively connected to an intraoral scanner forming part of said 3D scanner system. Some of the processors may be located inside the housing of the intraoral scanner, and other processors may be located on a computer forming part of the 3D scanner system.

### Parameterized model

The parameterized model may be a machine learning model, such as a trained neural network. A parameterized model may refer to a type of model, such as a machine learning model, that comprises a set of learnable parameters. These parameters may be values that the model adjusts during training to capture patterns, relationships, and/or features in the input data. Said input data may be captured two-dimensional images acquired by one or more camera units of the intraoral scanner.

The parameters of the parameterized model may be numerical values that define the behavior, such as the output, of the model. In the context of neural networks, the parameters may include weights and biases. The weights may be used to transform the input data, and the biases may be used to shift the transformed data. The value of these parameters may be learned from a training process, i.e. based on a training data set. During the training process, the model's parameters may be updated iteratively to minimize a specific loss function, which measures the difference between the model's predictions and the true target values. An optimization algorithm, e.g., based on gradient descent, may be utilized in the training process to adjust the parameters in order to improve the model's performance on the training data.

The parameterized model may be trained to approximate complex functions that map input data (e.g., images) to output predictions (e.g., position of pattern features). In other words, the model may learn to extract meaningful features from image(s) and use them to make predictions about the content of the image(s). The predictions may include the position of pattern features forming part of the spatial pattern in the images. Training of the parameterized model may in some cases be understood as adjusting or tuning the aforementioned parameters of the model, such that the output of the model is close to the true target values.

In some embodiments, the parameterized model is a neural network adapted or configured for solving or handling a translation-invariant problem. Generally, a translation-invariant problem in the context of computer vision refers to a scenario where the underlying task or objective remains the same despite spatial translations or shifts applied to the objects or features within the image. Typically, in translation-invariant problems, the exact position or location of objects in an image doesn't change the problem or its solution. Accordingly, the parameterized model of this disclosure may be trained or configured to recognize patterns and/or pattern features regardless of where they appear within the image. Thus, in some embodiments, the parameterized model or neural network is translation invariant such that the model or network produces the same response regardless of how its input is shifted.

Examples of suitable neural networks or architectures for handling translation-invariant problems include: Convolutional Neural Networks (CNNs), Siamese Networks, Triplet Networks, Capsule Networks (CapsNets), Attention-Based Models, Spatial Transformer Networks (STN), Region-based CNNs (R-CNNs), and Graph Neural Networks (GNNs). A convolutional neural network may utilize a plurality of convolutional layers, which enables the network to automatically learn and extract hierarchical features from the input data. The neural network may consist of layers of learnable parameters, including convolutional layers that extract features from images and fully connected layers that make predictions based on those features. Some examples of a suitable architecture of the neural network include: LeNet-5, AlexNet, VGG16, VGG19, GoogLeNet (Inception), ResNet, High-Resolution Net (HRNet), DenseNet, MobileNet, and EfficientNet. Other suitable neural networks and architectures can be envisaged without departing from the scope of the disclosure.

The architecture of the neural network may be configured to effectively process grid-like data, such as images. The architecture may comprise an input layer configured to receive input data, such as one or more images, each represented as a grid of pixels. The architecture may further comprise one or more convolutional layers, such as a plurality of convolutional layers. Each layer may be configured to apply a set of filters (kernels) to the input data to detect specific features (e.g., edges, textures, shapes) through convolution operations. In particular, it is desired to detect pattern features, such as corners, in the spatial pattern present in the images. The network architecture may further comprise one or more pooling layers, fully connected layers, and an output layer. The pooling layers may be placed subsequent to the convolutional layers, and they may have the purpose of reducing spatial dimensions and control overfitting. The fully connected layers may comprise a plurality of neurons, where every neuron is connected to every neuron in the previous and subsequent layers. These fully connected layers may combine extracted features and make predictions using learned weights and biases. The last fully connected layer may be configured to output predictions relevant to the specific task (e.g., position of pattern features in the spatial pattern in the images). The output layer may be configured to provide the final predictions based on the task (classification, regression).

### Training data set

The parameterized model or machine learning model may have been trained to suppress or ignore the part of the spatial pattern which has been reflected by the window and/or sleeve. This reflected part may also be referred to as artifacts in the images, since only the projected pattern on the scanned/imaged object is of desire, and not the direct reflection(s) from the window or sleeve. In order to achieve this purpose, the parameterized model or machine learning model may have been trained on the basis of a training data set.

The training data set may include ground truth data, such as the true position of the pattern features in the spatial pattern. The training data set may further include a plurality of two-dimensional training images comprising artifacts arising from reflections from the window and/or sleeve. The training data set may comprise a large number of said training images, such as at least 50 images, preferably at least 100 images, or even more preferably at least 250 images or more. The training images may have been acquired through the sleeve and/or window. Preferably, the images have been acquired such that there is no object in focus in the training images, except for the window and/or the sleeve. Advantageously, the training images comprises only the aforementioned artifacts and nothing else. Thus, when acquiring the training images, the background should preferably be entirely dark and/or such that no objects are in focus in the imaged scene. In some cases, the training images are obtained using the intraoral scanner disclosed herein. Thus, in some embodiments, the training data set includes ground truth data and the training images described herein above.

The training data set may further include two-dimensional rendered images of digital 3D models of teeth. The training data set may comprise a large number of said rendered images, such as at least 100 images, preferably at least 1.000 images, even more preferably at least 10.000 images. The digital 3D models may be 3D surface models. Preferably, the rendered images include at least a part of the spatial pattern. The digital 3D models may further include texture information of the three-dimensional scanned object, e.g., the color of the surface of the three-dimensional object. The rendered images may have been generated using a geometric virtual model of the intraoral scanner, wherein said geometric virtual model includes extrinsic and/or intrinsic parameters of the projector unit and camera units of the scanner. The training data set may further include data related to the pose(s) of the scanner used for generating a given digital 3D model, wherein each pose includes a position and/or orientation of the scanner. As an alternative to the rendered images of digital 3D models, the training data set may include real data e.g., obtained using the intraoral scanner disclosed herein. In the latter case, the data may be obtained without a window or sleeve on the scanner. In some cases, the ground truth data is generated based on the rendered images or the real data.

In some embodiments, the training data set includes, in addition to the ground truth data, two different sets of two-dimensional images, wherein a first set of images comprises artifacts arising from reflections from the window and/or sleeve, and wherein a second set of the images does not comprise said artifacts. The aforementioned training images may constitute the first set of images, and the rendered images of digital 3D models of teeth, or the real data obtained without window or sleeve, may constitute the second set of images. During the training phase of the parameterized model, the input to the model may be both sets of images, i.e., both the first set comprising artifacts and the second set without the artifacts. The input may further include the ground truth data, e.g., the position of the pattern features. Accordingly, the training data may comprise pairs of images, wherein each pair comprises a training image and a rendered image or a real image obtained without a hygienic barrier, such as a sleeve or window. In some embodiments, the number of training images is less than the number of rendered images. In that case, the same training image may be used for a plurality of rendered images, such that the number of pairs exceed e.g., 10.000 or even 100.000, even though the number of training images is far less, such as less than 1.000 or less than 100.

In some embodiments, a plurality of images are provided to the parameterized model or neural network as input, wherein the images are acquired at different moments in time. In that case, the parameterized model or neural network may be trained to take the past into account, such that previously acquired images are used as input to improve the output of the neural network.

### Detailed description of the drawings

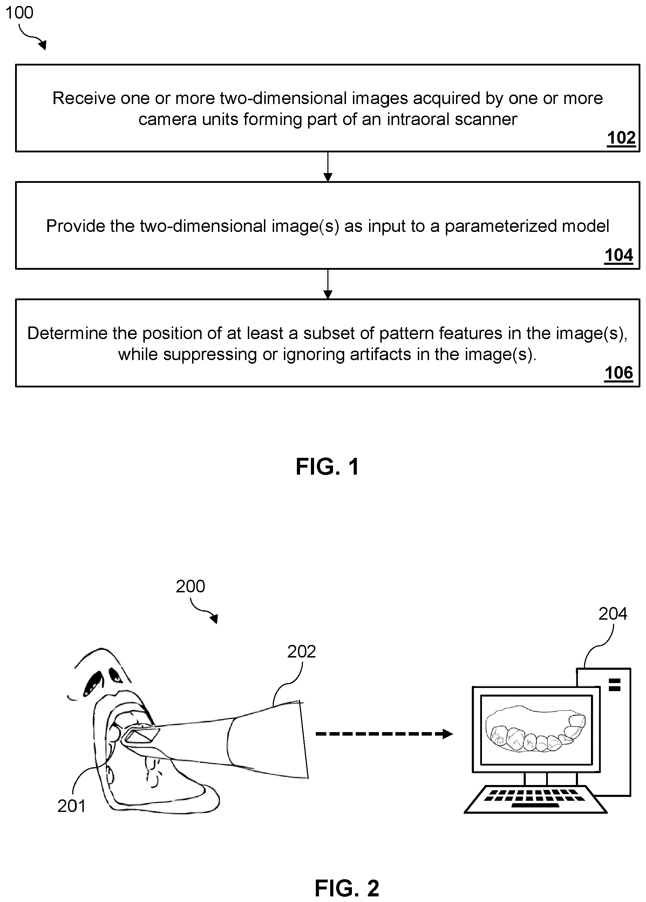

Fig. 1 shows a flowchart 100 according to an embodiment of the computer-implemented method disclosed herein. In step 102, one or more two-dimensional images are received, e.g., by one or more processors, wherein the images have been acquired by one or more camera units forming part of an intraoral scanner. Preferably, each image comprises at least a part of a spatial pattern comprising a plurality of pattern features. The image(s) may further comprise one or more artifacts arising from reflections from a window and/or sleeve of the intraoral scanner. In step 104, the two-dimensional image(s) are provided as input to a parameterized model. The parameters of the parameterized model may be set or chosen such that the artifacts are suppressed or ignored. In step 106, the positions of at least a subset of pattern features in the image(s) are determined, while suppressing or ignoring artifacts in the image(s).

Fig. 2 shows an embodiment of a 3D scanner system 200 according to the present disclosure. The 3D scanner system may be configured for generating a three-dimensional (3D) representation of an object 201, such as a dental object. As an example, the object 201 may be at least a part of the oral cavity including any of dentition, gingiva, retromolar trigone, hard palate, soft palate, and floor of the mouth, etc. In this embodiment, the 3D scanner system comprises an intraoral scanner 202 for acquiring a set of images of the scanned object, e.g. within the oral cavity of a person. The 3D scanner system further comprises one or more processors for generating a three-dimensional (3D) representation of the scanned object based on the acquired images. In general, the 3D representation may only represent a part of the object surface, e.g. captured by the field of view of the intraoral scanner 202. Such a 3D representation may also be referred to herein as a sub-scan or 3D surface. The processor(s) may be part of the 3D scanner 202, or they may be external to the intraoral scanner, or a combination of the two, i.e. such that some processing is performed on the 3D scanner, and further processing is performed on a computer system 204. The intraoral scanner may be configured to continuously, e.g., in real-time, acquire sets of images and generate one or more 3D surfaces and/or sub-scans based on said images. It may further be configured to continuously transmit, either wired or wirelessly, said sub-scans to a computer system 204. The sub-scans may be registered and stitched to each other to form a digital 3D model of the scanned object. Said 3D model may be displayed on a display, e.g. connected to the computer system.

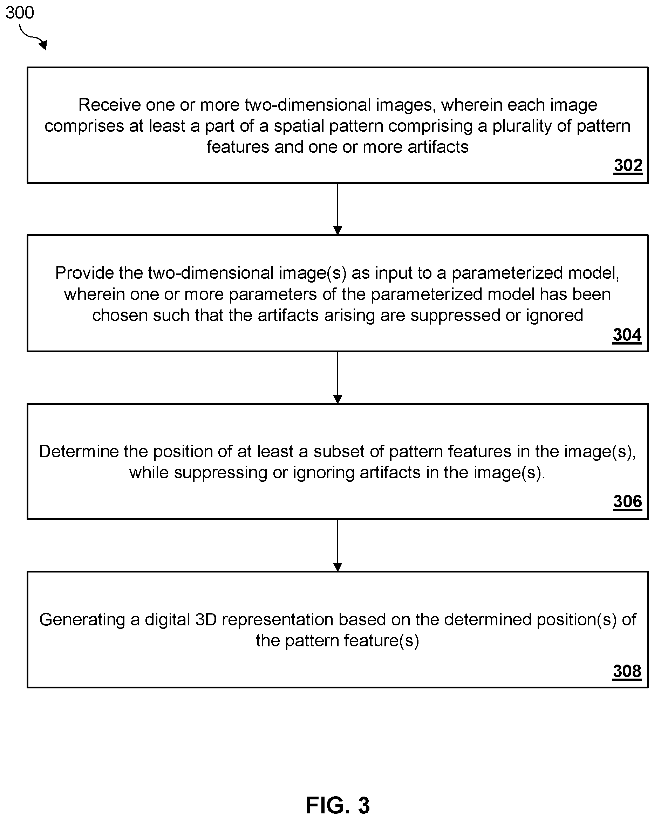

Fig. 3 shows a flowchart 300 according to an embodiment of the computer-implemented method disclosed herein. In step 302, one or more two-dimensional images are received, e.g., by the one or more processors of the 3D scanner system, wherein each image comprises at least a part of a spatial pattern comprising a plurality of pattern features and one or more artifacts. In step 304, the two-dimensional image(s) are provided as input to a parameterized model, wherein one or more parameters of the parameterized model has been chosen such that the artifacts arising are suppressed or ignored. In step 306, the position of at least a subset of pattern features in the image(s) is determined, while suppressing or ignoring artifacts in the image(s). In step 308, a digital 3D representation is generated based on the determined position(s) of the pattern feature(s).

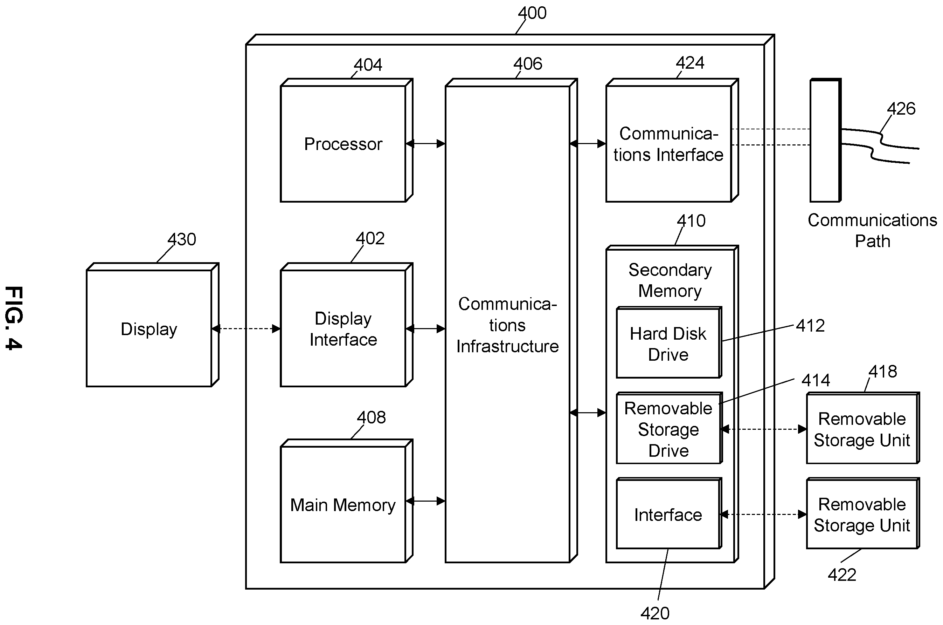

Fig. 4 shows a computer system 400 in which embodiments of the present disclosure, or portions thereof, may be implemented as computer-readable code. The computer system may encompass a number of components enabling data processing, storage, communication, and user interaction. The computer system described herein may comprise one or more processors 404, a communications interface 424, a hard disk drive 412, a removable storage drive 414, an interface 420, a main memory 408, a display interface 402, and a display 430.

The one or more processor(s) 404 may be configured for executing one or more steps of the computer-implemented methods disclosed herein. The communications interface 424 may be configured to allow the computer system to communicate with external devices and networks. It may support various communication protocols, such as Ethernet, Wi-Fi, or Bluetooth, enabling the exchange of data and facilitating connectivity with other devices. The hard disk drive 412 may be configured to provide non-volatile storage for the computer system. It may store software programs, operating system files, user data, and other files in a medium, such as a magnetic medium. The hard disk drive 412 may ensure persistent storage, allowing data to be retained even when the system is powered off. The removable storage drive 414, such as a CD/DVD drive or USB port, may be configured to provide the capability of the computer system to read from and/or write to a removable storage unit 418. This allows users to access external storage devices, such as optical discs or USB flash drives, and transfer data to and from the computer system. The interface 420 may be configured to connect various external devices, such as keyboards, mice, printers, scanners, or audio devices, to the computer system.

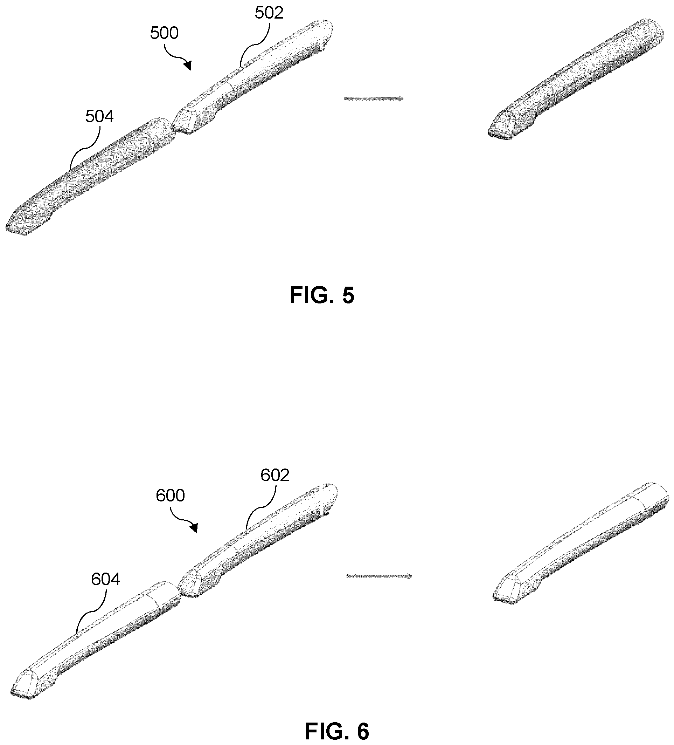

Fig. 5 shows an intraoral scanner 500 according to an embodiment of the present disclosure. The scanner 500 comprises an elongated housing 502. The scanner system further comprises a sleeve 504 adapted for being mounted on the outside of the elongated housing. In this embodiment, the sleeve 504 is a flexible transparent sleeve, such as a flexible polymer sleeve. The sleeve is adapted for ensuring a tight fit on the outside of the housing. The figure shows the scanner and sleeve before the sleeve is mounted (left) and after the sleeve is mounted (right). The housing comprises an aperture in a sidewall of the distal end of the housing (not visible in the figure), and the sleeve covers said aperture when mounted on the scanner.

Fig. 6 shows an intraoral scanner 600 according to an embodiment of the present disclosure. The scanner 600 comprises an elongated housing 602. The scanner system further comprises a sleeve 604 adapted for being mounted on the outside of the elongated housing. In this embodiment, the sleeve 604 is a rigid sleeve, such as manufactured by injection-molding. The figure shows the scanner and sleeve before the sleeve is mounted (left) and after the sleeve is mounted (right). The housing comprises an aperture in a sidewall of the distal end of the housing (not visible in the figure), and the sleeve covers said aperture when mounted on the scanner.

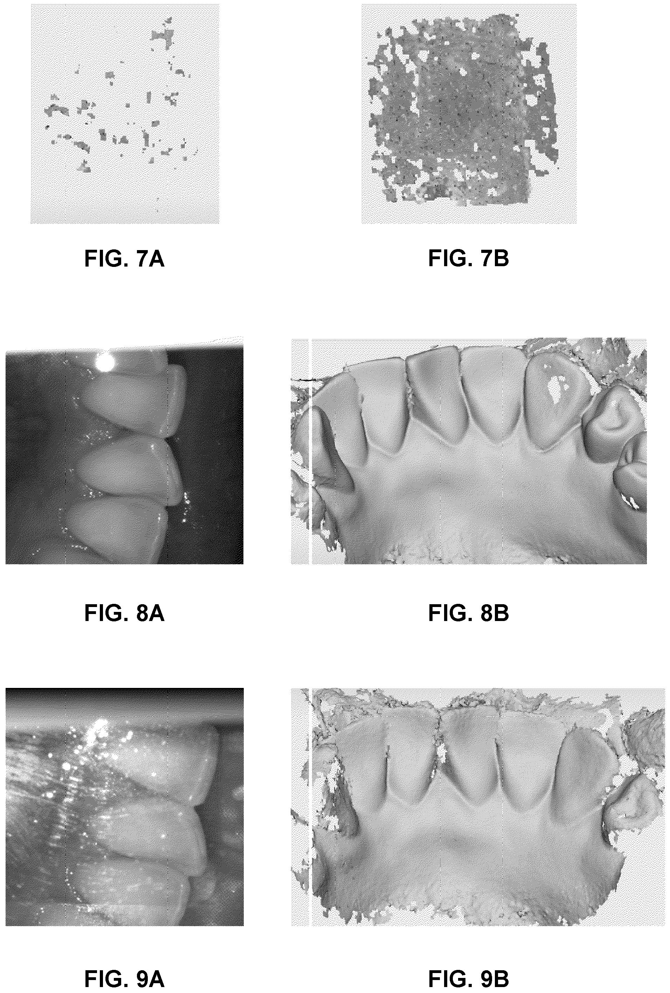

Fig. 7A shows a single sub-scan (3D representation) generated by the scanner in a case wherein a window and sleeve covers the aperture of the scanner housing, and wherein the parameterized model has only been trained to identify the position of pattern features, but it has not been trained to ignore or suppress the artifacts resulting from the window and/or sleeve. It is observed that the sub-scan is completely useless, since there is barely any visible 3D surface in the 3D representation of the teeth. Furthermore, the 3D scanner is not able to register further 3D data to the "model", since there are so few 3D points determined. Also, the 3D points may not even relate to the scanned surface of the teeth, but rather be artifacts, i.e., falsely determined 3D points belonging to the surface of the hygienic barrier (sleeve/window). The example serves to illustrate the complexity of scanning through a hygienic barrier, such as a transparent sleeve.

Fig. 7B shows a few subscans that are fused into a 3D model that resembles a noisy plane, or noisy displaced elements of a plane, which constitutes a noisy scan of the window and/or sleeve surface itself. Thus, this 3D model cannot be used for anything, since it does not represent the surface of the intended scanned object, i.e., the dentition of the patient. The example serves to illustrate the complexity of scanning through a hygienic barrier, such as a transparent sleeve.

Fig. 8A shows a raw image of some teeth, wherein the image has been captured through a window arranged in the aperture of the housing of an intraoral scanner. A bright spot (white) is visible in the top of the image, said bright spot originating from a specular reflection of the window of the scanner. The image of fig. 8A corresponds to a single field-of-view of the scanner. There was no sleeve covering the window during the capture of this image.

Fig. 8B shows a digital 3D model of the same teeth shown in fig. 8A, wherein the 3D model has been generated based on a plurality of captured images of the teeth. Thus, the model has been generated from a plurality of views, where the image of fig. 8A illustrates one of said views. In this case, there was no sleeve covering the window during the capture of the images; thus the corresponding 3D model appears smooth and accurate with high quality.

Fig. 9A shows a raw image of some teeth, wherein the image has been captured through a window and a sleeve, wherein the sleeve covers the window, and wherein the window is located in the optical path of an intraoral scanner. Multiple bright spots (white circles) are visible in the raw image, said bright spots formed due to e.g. the projector light being reflected by the many wrinkles in the sleeve. The bright spots complicates the image processing; in particular it makes it difficult to identify and determine pattern features in the spatial pattern present on the teeth. Consequently, this complicates the determination of 3D points, which negatively affects the accuracy of the generated 3D model. A plurality of images similar to the one in fig. 9A are input to a trained parameterized model for determining pattern features in the images while suppressing or ignoring artifacts from the window and/or sleeve.

Fig. 9B shows a rendition of the digital 3D model, where the images have been captured through both a window and a sleeve covering the window (similar to the raw image shown in fig. 9A), wherein the images have been input to a trained parameterized model as described herein. It is observed that even though the 3D model appears with more surface roughness, the scanner system is still able to utilize the otherwise poor image input (as evident by fig. 9A) due to the trained parameterized model.

## Claims

1. A 3D scanner system comprising:
- an intraoral scanner (202; 500; 600) comprising:
- an elongated housing (502; 602) comprising a distal end for being inserted into an oral cavity, wherein the housing (502; 602) comprises an aperture in a sidewall of the distal end of the housing (502; 602);
- a window arranged in the aperture of the housing (502; 602); and/or
- a sleeve (504; 604) mounted on the outside of the elongated housing (502; 602), wherein the sleeve (504; 604) covers the aperture;
- a projector unit configured to project a predefined spatial pattern through the aperture and onto at least a part of the surface of a three-dimensional object (201), wherein the spatial pattern comprises a plurality of pattern features; and
- one or more camera units configured to acquire one or more two-dimensional images of the three-dimensional object (201), wherein at least a part of the spatial pattern is present in the images; and
- one or more processors (404) operatively connected to the intraoral scanner (202; 500; 600), said processors (404) configured to:
- receive the one or more two-dimensional images comprising a plurality of pattern features, wherein the images comprises one or more artifacts arising from reflections of the spatial pattern from the window and/or sleeve (504; 604);
**CHARACTERIZED IN THAT** said processors (404) are further configured to:
- provide the two-dimensional image(s) as input to a parameterized model trained to determine the position of at least a subset of the pattern features in the image(s), while suppressing or ignoring the artifacts; and
- generating a digital 3D representation of the scanned object based on the determined position(s) of the pattern feature(s).

2. The 3D scanner system according to claim 1, wherein one or more parameters of the parameterized model have been chosen or adjusted such that the artifacts are suppressed or ignored by the parameterized model.

3. The 3D scanner system according to any of the preceding claims, wherein the parameterized model is a machine learning model, such as a neural network.

4. The 3D scanner system according to any of the preceding claims, wherein the parameterized model has been trained on the basis of a training data set including two different sets of two-dimensional images, wherein a first set of the images comprises artifacts arising from reflections from the window and/or sleeve, and a second set of the images does not comprise said artifacts.

5. The 3D scanner system according to claim 4, wherein the training data set further includes ground truth data, said ground truth data including the position of the pattern features.

6. The 3D scanner system according to any of the claims 4-5, wherein the training data set includes the ground truth data and two-dimensional training images comprising artifacts arising from reflections from the window and/or sleeve, wherein said training images have been acquired through the window and/or sleeve, wherein there is no object in focus except for the window and/or the sleeve.

7. The 3D scanner system according to any of the claims 4-6, wherein the training data set further includes two-dimensional rendered images of digital 3D models of teeth, wherein the rendered images have been generated using a geometric virtual model of the intraoral scanner.

8. The 3D scanner system according to any of the preceding claims, wherein the parameterized model or neural network is translation invariant such that the neural network produces the same response regardless of how its input is shifted.

## Patentansprüche

1. 3D-Scannersystem, umfassend:
- einen intraoralen Scanner (202; 500; 600), umfassend:
- ein längliches Gehäuse (502; 602), das ein distales Ende umfasst, um in einen oralen Hohlraum eingeführt zu werden, wobei das Gehäuse (502; 602) eine Öffnung in einer Seitenwand des distalen Endes des Gehäuses (502; 602) umfasst;
- ein Fenster, das in der Öffnung des Gehäuses (502; 602) angeordnet ist; und/oder
- eine Hülse (504; 604), die an der Außenseite des länglichen Gehäuses (502; 602) montiert ist, wobei die Hülse (504; 604) die Öffnung abdeckt;
- eine Projektoreinheit, die dazu konfiguriert ist, ein vordefiniertes räumliches Muster durch die Öffnung und auf mindestens einen Teil der Oberfläche eines dreidimensionalen Objekts (201) zu projizieren, wobei das räumliche Muster eine Vielzahl von Mustermerkmalen umfasst; und
- eine oder mehrere Kameraeinheiten, die dazu konfiguriert ist, ein oder mehrere zweidimensionale Bilder des dreidimensionalen Objekts (201) zu erfassen, wobei mindestens ein Teil des räumlichen Musters in den Bildern vorhanden ist; und
- einen oder mehrere Prozessoren (404), die mit dem intraoralen Scanner (202; 500; 600) wirkverbunden sind, wobei die Prozessoren (404) zu Folgendem konfiguriert sind:
- Empfangen des einen oder der mehreren zweidimensionalen Bilder, die eine Vielzahl von Mustermerkmalen umfassen, wobei die Bilder ein oder mehrere Artefakte umfassen, die aus Reflexionen des räumlichen Musters von dem Fenster und/oder der Hülse (504; 604) entstehen;
**DADURCH GEKENNZEICHNET, DASS** die Prozessoren (404) ferner zu Folgendem konfiguriert sind:
- Bereitstellen des/der zweidimensionalen Bildes/Bilder als Eingabe in ein parametrisiertes Modell, das trainiert ist, um die Position von mindestens einem Teilsatz der Mustermerkmale in dem/den Bild(ern) zu bestimmen, während die Artefakte unterdrückt oder ignoriert werden; und
- Erzeugen einer digitalen 3D-Darstellung des gescannten Objekts auf Grundlage der bestimmten Position(en) des/der Mustermerkmals/Mustermerkmale.

2. 3D-Scannersystem nach Anspruch 1, wobei ein oder mehrere Parameter des parametrisierten Modells derart ausgewählt oder eingestellt wurden, dass die Artefakte von dem parametrisierten Modell unterdrückt oder ignoriert werden.

3. 3D-Scannersystem nach einem der vorhergehenden Ansprüche, wobei das parametrisierte Modell ein Modell für maschinelles Lernen ist, wie etwa ein neuronales Netz.

4. 3D-Scannersystem nach einem der vorhergehenden Ansprüche, wobei das parametrisierte Modell auf Grundlage eines Trainingsdatensatzes trainiert wurde, der zwei verschiedene Sätze von zweidimensionalen Bildern beinhaltet, wobei ein erster Satz der Bilder Artefakte umfasst, die aus Reflexionen von dem Fenster und/oder der Hülse entstehen, und ein zweiter Satz der Bilder die Artefakte nicht umfasst.

5. 3D-Scannersystem nach Anspruch 4, wobei der Trainingsdatensatz ferner Ground-Truth-Daten beinhaltet, wobei die Ground-Truth-Daten die Position der Mustermerkmale beinhalten.

6. 3D-Scannersystem nach einem der Ansprüche 4-5, wobei der Trainingsdatensatz die Ground-Truth-Daten und zweidimensionale Trainingsbilder beinhaltet, die Artefakte umfassen, die aus Reflexionen von dem Fenster und/oder der Hülse entstehen, wobei die Trainingsbilder durch das Fenster und/oder die Hülse erfasst wurden, wobei abgesehen von dem Fenster und/oder der Hülse kein Objekt im Fokus ist.

7. 3D-Scannersystem nach einem der Ansprüche 4-6, wobei der Trainingsdatensatz ferner zweidimensionale gerenderte Bilder von digitalen 3D-Modellen von Zähnen beinhaltet, wobei die gerenderten Bilder unter Verwendung eines geometrischen virtuellen Modells des intraoralen Scanners erzeugt wurden.

8. 3D-Scannersystem nach einem der vorhergehenden Ansprüche, wobei das parametrisierte Modell oder das neuronale Netz translationsinvariant ist, sodass das neuronale Netz die gleiche Antwort unabhängig davon, wie seine Eingabe verschoben wird, erstellt.

## Revendications

1. Système de scanner 3D comprenant :
- un scanner intraoral (202 ; 500 ; 600) comprenant :
- un boîtier allongé (502 ; 602) comprenant une extrémité distale destinée à être insérée dans une cavité buccale, dans lequel le boîtier (502 ; 602) comprend une ouverture dans une paroi latérale de l'extrémité distale du boîtier (502 ; 602) ;
- une fenêtre agencée dans l'ouverture du boîtier (502 ; 602) ; et/ou
- un manchon (504 ; 604) monté sur l'extérieur du boîtier allongé (502 ; 602), dans lequel le manchon (504 ; 604) recouvre l'ouverture ;
- une unité de projecteur configurée pour projeter un motif spatial prédéfini à travers l'ouverture et sur au moins une partie de la surface d'un objet tridimensionnel (201), dans lequel le motif spatial comprend une pluralité de caractéristiques de motif ; et
- une ou plusieurs unités de caméra configurées pour acquérir une ou plusieurs images bidimensionnelles de l'objet tridimensionnel (201), dans lequel au moins une partie du motif spatial est présente dans les images ; et
- un ou plusieurs processeurs (404) connectés de manière opérationnelle au scanner intraoral (202 ; 500 ; 600), lesdits processeurs (404) étant configurés pour :
- recevoir les une ou plusieurs images bidimensionnelles comprenant une pluralité de caractéristiques de motif, dans lequel les images comprennent un ou plusieurs artefacts provenant de réflexions du motif spatial provenant de la fenêtre et/ou du manchon (504 ; 604) ;
**CARACTERISE EN CE QUE** lesdits processeurs (404) sont en outre configurés pour :
- fournir l'image ou les images bidimensionnelles en tant qu'entrée à un modèle paramétré entraîné pour déterminer la position d'au moins un sous-ensemble des caractéristiques de motif dans l'image ou les images, tout en supprimant ou en ignorant les artefacts ; et
- générer une représentation 3D numérique de l'objet scanné sur la base de l'une ou plusieurs positions déterminées de l'une ou plusieurs caractéristiques de motif.

2. Système de scanner 3D selon la revendication 1, dans lequel un ou plusieurs paramètres du modèle paramétré ont été choisis ou ajustés de sorte que les artefacts soient supprimés ou ignorés par le modèle paramétré.

3. Système de scanner 3D selon l'une quelconque des revendications précédentes, dans lequel le modèle paramétré est un modèle d'apprentissage automatique, tel qu'un réseau de neurones.

4. Système de scanner 3D selon l'une quelconque des revendications précédentes, dans lequel le modèle paramétré a été entraîné sur la base d'un ensemble de données d'entraînement incluant deux ensembles différents d'images bidimensionnelles, dans lequel un premier ensemble des images comprend des artefacts provenant de réflexions provenant de la fenêtre et/ou du manchon, et un second ensemble des images ne comprend pas lesdits artefacts.

5. Système de scanner 3D selon la revendication 4, dans lequel l'ensemble de données d'entraînement inclut en outre des données de vérité terrain, lesdites données de vérité terrain incluant la position des caractéristiques de motif.

6. Système de scanner 3D selon l'une quelconque des revendications 4 à 5, dans lequel l'ensemble de données d'entraînement inclut les données de vérité terrain et des images d'entraînement bidimensionnelles comprenant des artefacts provenant de réflexions provenant de la fenêtre et/ou du manchon, dans lequel lesdites images d'entraînement ont été acquises à travers la fenêtre et/ou le manchon, dans lequel il n'y a aucun objet au foyer à l'exception de la fenêtre et/ou du manchon.

7. Système de scanner 3D selon l'une quelconque des revendications 4 à 6, dans lequel l'ensemble de données d'entraînement inclut en outre des images rendues bidimensionnelles de modèles 3D numériques de dents, dans lequel les images rendues ont été générées à l'aide d'un modèle virtuel géométrique du scanner intraoral.

8. Système de scanner 3D selon l'une quelconque des revendications précédentes, dans lequel le modèle paramétré ou le réseau de neurones est invariant par translation de sorte que le réseau de neurones produit la même réponse indépendamment de la manière dont son entrée est décalée.
